# EUROPÄISCHE PATENTSCHRIFT

(11) **EP 0 604 364 B1**
(45) Veröffentlichungstag und Bekanntmachung des Hinweises auf die Patenterteilung: **25.02.1998**
(21) Anmeldenummer: 93810871.9
(22) Anmeldetag: 13.12.1993
(51) Int. Cl.: C08G 59/17, C07D 303/12, C08L 63/10, G03F 7/038

(54) **Neue (cyclo)aliphatische Epoxidverbindungen**
New (cyclo)aliphatic epoxy compounds
Composés époxy (cyclo)aliphatiques nouveaux

(30) Priorität: 23.12.1992 CH 3943/92
(43) Veröffentlichungstag der Anmeldung: 29.06.1994
(73) Patentinhaber: Ciba Specialty Chemicals Holding Inc., 4057 Basel (CH)
(72) Erfinder: Steinmann, Bettina, Dr., CH-1724 Praroman (CH); Wolf, Jean-Pierre, Dr., CH-1791 Courtaman (CH); Schulthess, Adrian, Dr., CH-1734 Tentlingen (CH); Hunziker, Max, Dr., CH-3186 Düdingen (CH)

(56) Entgegenhaltungen:
- EP-A- 0 282 834
- EP-A- 0 485 990
- DE-A- 4 232 213
- GB-A- 788 531

## Beschreibung

Die vorliegende Erfindung betrifft neue (cyclo)aliphatische Epoxidverbindungen, deren Herstellung und Verfahren zum Polymerisieren dieser Verbindungen mittels aktinischer Bestrahlung und deren Einsatz z.B. in der Stereolithographie zur Herstellung von dreidimensionalen Gegenständen sowie die Verwendung der neuen (cyclo)aliphatischen Epoxidverbindungen z.B. als Klebstoffe, Beschichtungen und Resists.

Bekanntlich können strahlungsempfindliche Harze oder Harzgemische vielseitig angewendet werden, beispielsweise als Beschichtungsmittel, Klebstoffe oder Photoresists. Im Prinzip sollten diese Harze oder Harzsysteme generell auch für die Herstellung von dreidimensionalen Gegenständen (3D-Objekten) nach dem im US-Patent 4,575,330 beschriebenen stereolithographischen Verfahren geeignet sein, doch erweisen sich viele Harze als zu viskos, und andere wiederum sind zu wenig lichtempfindlich oder unterliegen beim Härten einem zu grossen Schrumpfungsprozess. Auch lassen die Festigkeitseigenschaften der Formkörper bzw. Gegenstände aus photogehärteten Harzen oft zu wünschen übrig.

Mittels des stereolithographischen Verfahrens kann man bekanntlich komplizierte dreidimensionale Gegenstände aus flüssigen, lichtempfindlichen Harzen herstellen. Solche Gegenstände werden schichtweise aufgebaut, wobei jede neue härtbare Harzschicht durch Vorhärtung mittels UV/VIS-Licht mit der vorhergehenden, vorgehärteten Schicht fest verbunden wird. Der Gesamtaufbau des dreidimensionalen Gegenstandes lässt sich bekanntlich nach einem computergesteuerten Prozess bewerkstelligen.

In den letzten Jahren hat es nicht an Versuchen gefehlt, Harzsysteme zu entwickeln, welche in stereolithographischen Verfahren einsetzbar sind. H. Kodama offenbart in Rev. Sci. Instrum. **52** (11), 1170-1173 (1981) unter dem Handelsnamen "Tevista" ein flüssiges, photohärtbares Harzgemisch bestehend aus einem ungesättigten Polyester, einem Acrylsäureester, Styrol, einem Polymerisationsinitiator und einem Sensibilisator. Dieses Harzsystem ist für das stereolithographische Verfahren jedoch mit dem Nachteil behaftet, dass die Photoempfindlichkeit ungenügend ist und die sogenannte "Grünfestigkeit" (green strength) der mit Laserstrahlen vorgehärteten Gegenstände relativ niedrig ist.

Im US-Patent 4,575,330 wird ein stereolithographisches Verfahren vorgeschlagen, wonach als flüssiges Harz ein modifiziertes Acrylat eingesetzt wird, das in der Beschreibung als "Potting Compound 363" bezeichnet wird. Solche Harzgemische werden im US-Patent 4,100,141 offenbart. Auch sie haben den Nachteil, dass sie ungenügend photoempfindlich sind und lange Zeiten für die Herstellung von dreidimensionalen Gegenständen nach dem stereolithographischen Verfahren benötigen.

Daher ist es verständlich, dass an die Harze, die in stereolithographischen Verfahren eingesetzt werden sollen, hohe Anforderungen gestellt werden. Beispielsweise soll die Photoempfindlichkeit des Harzsystems so beschaffen sein, dass das Verhältnis von aufgewendeter Strahlungsenergie und erzielter Eindringtiefe in das flüssige photoempfindliche Harzgemisch, wobei die betreffenden Teile sich verfestigen, im vertretbaren Rahmen liegt Das heisst, bei einem für das stereolithographische Verfahren geeigneten Harz oder Harzgemisch soll mit wenig Strahlungsenergie eine möglichst hohe Härtungstiefe bei gleichzeitig hohem Polymerisationsgrad und guter Grünfestigkeit erreicht werden.

Beim Verfahren der aufeinanderfolgenden Polymerisation von dünnen Schichten, wie es im stereolithographischen Verfahren angewendet wird, ist gewöhnlich keine dieser Schichten völlig ausgehärtet. Der nicht völlig gehärtete Gegenstand wird als Grünling bezeichnet, und den Elastizitätsmodul und die Bruchfestigkeit dieses Grünlings bezeichnet man auch als Grünfestigkeit. Normalerweise wird der Grünling dann mit UV/VIS-Licht gehärtet, beispielsweise mittels einer Quecksilber- oder Xenonbogenlampe. Die Grünfestigkeit eines Werkstückes stellt daher einen wichtigen Parameter dar, da Gegenstände mit einer geringen Grünfestigkeit sich unter ihrem eigenen Gewicht deformieren können, oder sie können sich bei der Aushärtung durchbiegen oder sie sacken zusammen.

Gemäss dem Stand der Technik werden, um brauchbare Verbindungen vorallem für die Stereolithographie zu haben, u.a. auch in der EP-A-0 360 869 Mischungen von Acrylatverbindungen mit Epoxidharzen vorgeschlagen. Diese haben jedoch den Nachteil, dass sie spröde Endprodukte ergeben. Ausserdem entstehen bei der Härtung zwei unabhängige Netzwerke, was sich nachteilig auf die Grünfestigkeit auswirkt.

Schliesslich werden in der EP-Patentanmeldung Nr. 0485990 hitzehärtbare Zusammensetzungen beschrieben, welche Acrylpolyme mit funktionellen alicyclischen Epoxidgruppen enthalten.

Aufgabe der Erfindung war es daher, Verbindungen zu entwickeln, die die aufgezeigten Nachteile überwinden, d.h. für stereolithographische Anwendungen die richtige Viskosität und Lichtempfindlichkeit aufweisen, und welche kein unabhängiges Netzwerk, sondern ein zusammenhängendes, homogenes Netzwerk enthalten, so dass die Grünfestigkeit beziehungsweise die Festigkeitseigenschaften der Formkörper generell verbessert werden.

Gelöst wurde diese Aufgabe durch eine neue Klasse von (cyclo)aliphatischen Epoxidverbindungen. Diese enthalten in ein- und demselben Molekül mindestens eine Acrylat- und Epoxidgruppe, insbesondere je 2 oder auch mehr und stellen demnach multifunktionelle Verbindungen dar. Derartige Verbindungen sind im Stand der Technik nicht bekannt.

Es ist überraschend, dass einerseits solche Moleküle relativ problemlos hergestellt werden können, dass diese leicht radikalisch und/oder kationisch polymerisieren und andererseits, dass diese Verbindungen z.B. in der Stereolithographie gut einsetzbar sind. Dadurch erhält man ein zusammenhängendes, homogenes Netzwerk, was vorallem für die Grünfestigkeit Vorteile bietet.

Durch die Bestrahlung der aus diesen neuen (cyclo)aliphatischen Epoxidverbindungen hergestellten Formulierungen können somit unterschiedlich hohe Vernetzungsdichten erreicht werden, was zur Folge hat, dass sich sowohl die bei der Vorhärtung mittels Laserstrahlen entstehenden Grünlinge als auch die durch Aushärtung der Grünlinge erhaltenen Gegenstände durch in weiten Grenzen variierbare und vor allem gute Eigenschaften, in erster Linie gute Festigkeitseigenschaften, auszeichnen.

Gegenstand vorliegender Erfindung sind somit neue (cyclo)aliphatische Epoxidverbindungen der Formeln 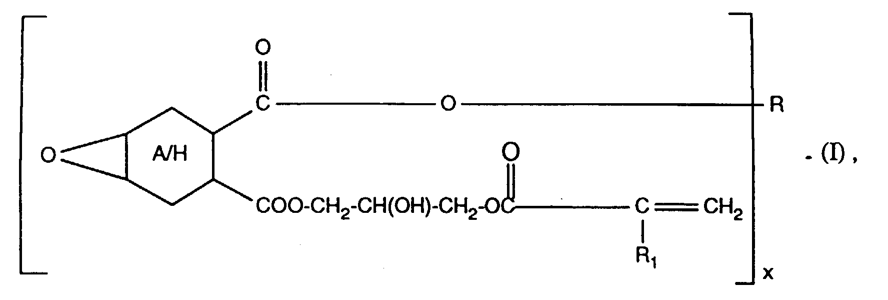 und 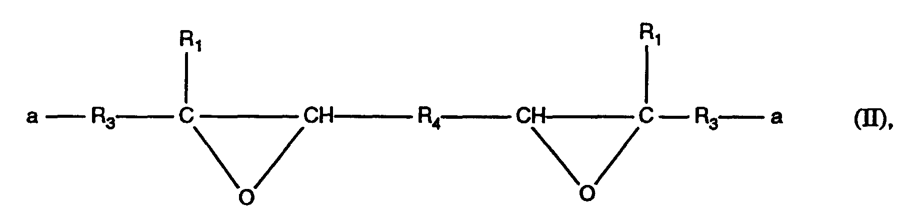 worin
a einen Rest der Formel 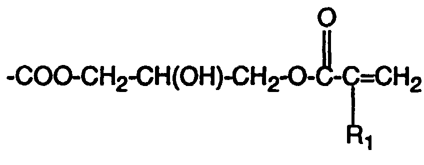 bedeutet, A einen hydrierten Benzolrest (Cyclohexylrest) oder einen hydrierten Rest der Formel 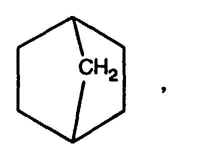 R einen Rest der Formeln (-CH₂)_{y}CH₃, (-CH₂-)y, 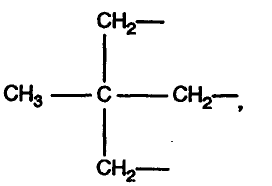 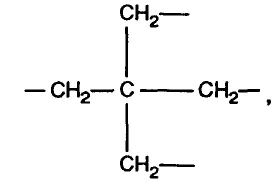 einen unsubstituierten oder substituierten 1 bis 6-wertigen aliphatischen Alkoholrest, einen unsubstituierten oder substituierten aromatisch-aliphatischen Alkoholrest, einen unsubstituierten oder substituierten 1- bis 4-wertigen Polyetherrest oder Polyesterrest, einen unsubstituierten oder substituierten 1- bis 4-wertigen Polycaprolactonrest, einen unsubstituierten oder substituierten 1- bis 4-wertigen Polyurethanrest oder einen Rest der Formel 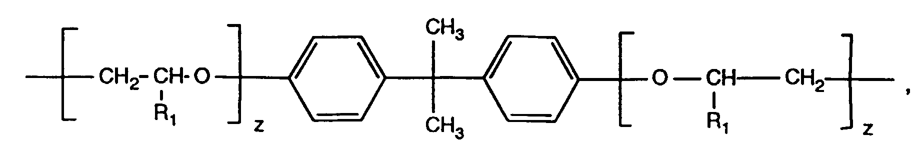 und
R₁ Wasserstoff oder CH₃ bedeuten, wobei
x eine ganze Zahl von 1 bis 6,
y eine ganze Zahl von 2 bis 20,
z eine ganze Zahl von 1 bis 10, und
R₃ und R₄ unabhängig voneinander einen unsubstituierten oder substituierten aliphatischen, aromatischen oder cycloaliphatischen Kohlenwasserstoffrest bedeuten, wobei es sich im Falle eines unsubstituierten oder substituierten aliphatischen Kohlenwasserstoffrests um eine gerade oder verzweigte C₁-C₂₀-Alkylkette handelt, welche ein- oder mehrmals substituiert sein kann und welche auch durch Ester- oder Ether-Gruppierungen unterbrochen sein kann.

Bevorzugt sind Verbindungen der Formel I, insbesondere Verbindungen der Formel I, in welcher A einen hydrierten Benzolrest, den Rest R[-CH₂-]_{y} oder einen Rest der Formel 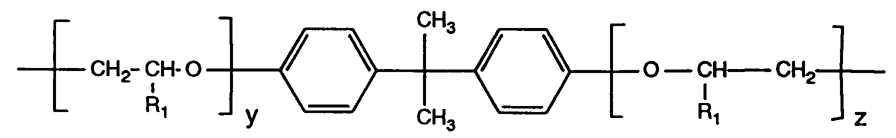 bedeuten, wobei y, z und R₁ die im Anspruch 1 angegebene Bedeutung haben und x 2 bedeutet.

Bedeutet R die Reste (-CH₂)_{y}CH₃ oder (-CH₂-)_{y}, so können diese Alkyl- bzw. Alkylenreste geradkettig oder verzweigt sein. Beispielsweise handelt es sich dabei um Methyl, Ethyl, n-Propyl, Isopropyl, n-Butyl, sec.-Butyl, tert.-Butyl, Pentyl, Hexyl, Heptyl, Octyl, Nonyl, Decyl, Undedyl, Dodecyl, Tetradecyl, Hexadecyl, Icosyl oder Docosyl beziehungsweise um die entsprechenden Alkylenreste.

Bedeutet R einen unsubstituierten oder substituierten 1 bis 6-wertigen aliphatischen Alkoholrest, so kommen vorallem die Reste folgender Verbindungen in Frage: aliphatische Diole, wie Ethylenglykol, Propan-1,2-diol, Propan-1,3-diol, Butan-1,4-diol, Pentan-1,5-diol, Neopentylglykol, Hexan-1,6-diol, Octan-1,8-diol, Decan-1,10-diol oder Dodecan-1,12-diol; cycloaliphatische Diole, wie 1,3- oder 1,4-Dihydroxycyclohexan, 1,4-Cyclohexandimethanol, Bis-(4-hydroxycyclohexyl)-methan oder 2,2-Bis-(4-hydroxycyclohexyl)-propan. Beispiele für höherfunktionelle Alkohole sind 1,1,1-Trimethylolethan, 1,1,1-Trimethylolpropan, Glycerin, Pentaerithrit oder 1,3,5-Trihydroxybenzol.

Bedeutet R einen unsubstituierten oder substituierten aromatisch-aliphatischen Alkoholrest, so handelt es sich dabei beispielsweise um die Reste folgender Verbindungen:
Benzylalkohol, Phenylethylalkohol, Phenylpropylalkohol und um die Verbindung der Formel

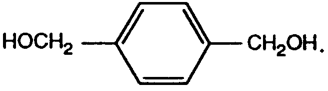

Bedeutet R einen unsubstituierten oder substituierten 1- bis 4-wertigen Polyetherrest, so kommen vorallem folgende Reste in Frage: 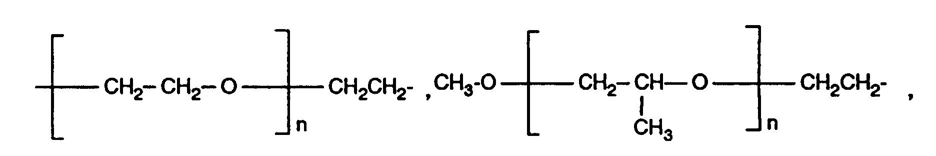 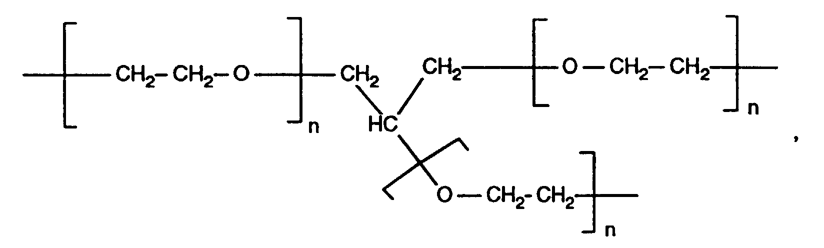 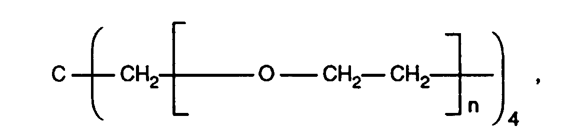 wobei n eine ganze Zahl von 1 bis 20 bedeutet, oder 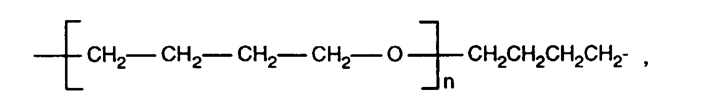 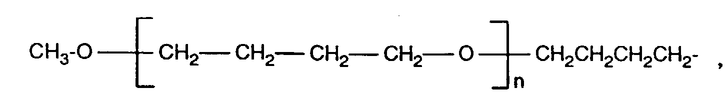 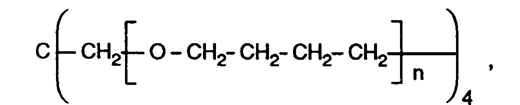 wobei n eine ganze Zahl von 1 bis 20 bedeutet.

Bedeutet R einen unsubstituierten oder substituierten 1- bis 4-wertigen Polyesterrest, so handelt es sich dabei z.B. um folgende Reste: 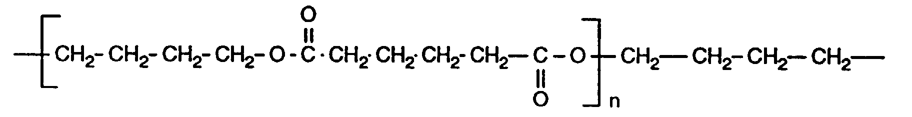 und 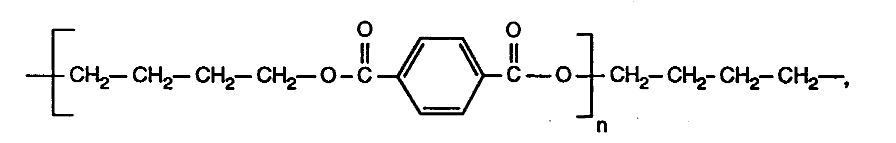 wobei n eine ganze Zahl von 1 bis 20 bedeutet.

Bedeutet R einen unsubstituierten oder substituierten 1- bis 4-wertigen Polycaprolactonrest, so handelt es sich dabei z.B. um folgende Reste: 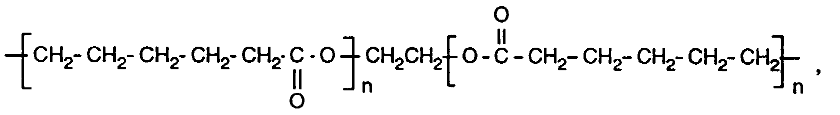 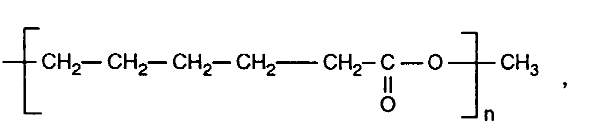 wobei n eine ganze Zahl von 1 bis 20 bedeutet.

Bedeutet R einen unsubstituierten oder substituierten 1- bis 4-wertigen Polyurethanrest, so handelt es sich dabei z.B. um folgende Reste: 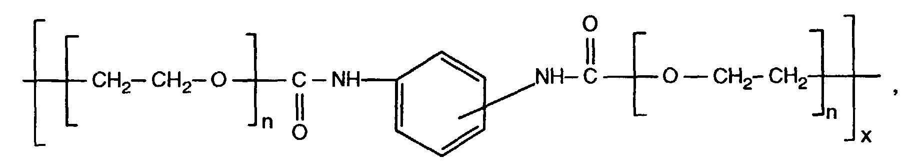 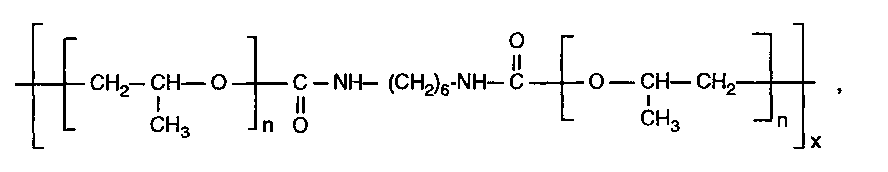 worin n eine ganze Zahl von 1 bis 20 und x eine ganze Zahl von 1 bis 10 bedeuten.

Bedeuten R₃ und R₄ einen unsubstituierten oder substituierten aliphatischen Kohlenwasserstoffrest, so handelt es sich dabei um eine gerade oder verzweigte C₁-C₂₀-Alkylenkette, welche ein- oder mehrmals substituiert sein kann, z.B. durch Halogen, wie F, Cl oder Br, durch NO₂, CN oder Aryl, wie Phenyl, und welche auch durch Ester oder Ether Gruppierungen unterbrochen sein kann; beispielsweise handelt es sich dabei um die folgenden Reste:
-(CH₂)₂-, -(CH₂)₆-, Isopropyl, Isobutyl oder auch um einen (Poly)-Ether- oder (Poly)-Esterrest, z.B. 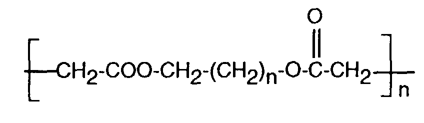 oder 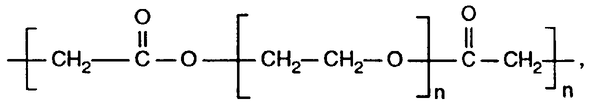 worin n eine ganze Zahl von 1 bis 20 bedeutet.

Bedeuten R₃ und R₄ einen unsubstituierten oder substituierten aromatischen Kohlenwasserstoffrest, so handelt es sich z.B. um einen Phenyl- oder Naphthylrest, der durch einen C₁-C₂₀ Alkylrest substituiert sein kann, wobei der Alkylrest ein- oder mehrmals substituiert sein kann, z.B. durch Halogen, wie F, Cl oder Br, durch NO₂, CN oder Aryl, wie Phenyl, und auch durch Ester oder Ether Gruppierungen unterbrochen sein kann; beispielsweise handelt es sich dabei um die folgenden Reste:

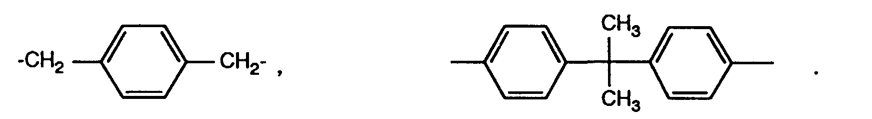

Bedeuten R₃ und R₄ einen unsubstituierten oder substituierten cycloaliphatischen Kohlenwasserstoffrest, so handelt es sich dabei z.B. um cycloaliphatische 5-, 6- oder 7-Ringe, die durch einen C₁-C₂₀ Alkylrest substituiert sein können, wobei der Alkylrest ein- oder mehrmals substituiert sein kann, z.B. durch Halogen, wie F, Cl oder Br, durch NO₂, CN oder Aryl, wie Phenyl, und auch durch Ester oder Ether Gruppierungen unterbrochen sein kann; beispielsweise handelt es sich dabei um die folgenden Reste:

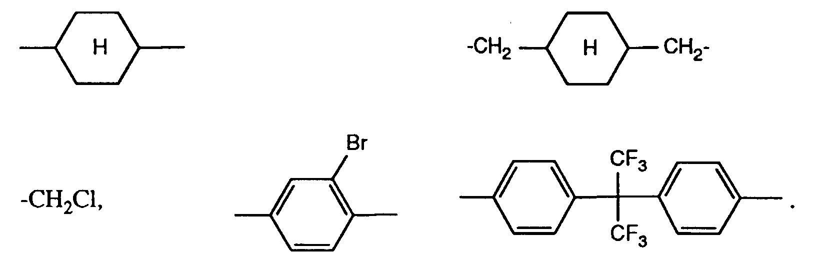

Die erfindungsgemässen (cyclo)aliphatischen Epoxidverbindungen sind in der Regel und je nach Art der Reste R, beziehungsweise R₃ und R₄, nieder- bis hochviskose Harze, die sich leicht in organischen Lösungsmitteln, wie Toluol, Essigester, Tetrahydrofuran sowie in anderen polymerisierbaren Monomeren, wie (Meth-)Acrylaten oder Epoxiden, lösen.

Die (cyclo)aliphatischen Epoxidverbindungen der Formel I können in an sich bekannter Weise hergestellt werden. Z.B. erfolgt die Herstellung der Verbindungen der Formel I dadurch, dass mindestens 1 Mol eines cyclischen, ungesättigten Anhydrides der Formel III 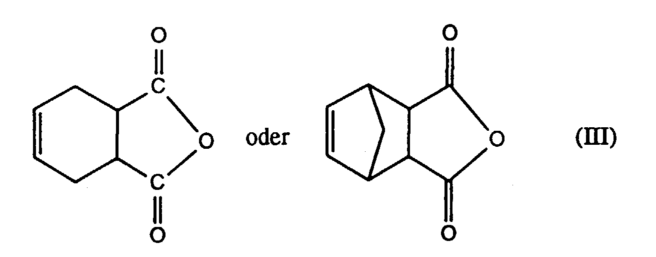 mit mindestens 1 Mol einer den Rest R einführenden Verbindung zu einer Diester-Disäure der Formel IV 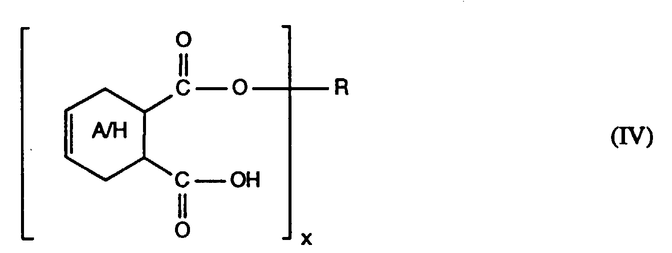 umgesetzt wird, welche dann mit Glycidyl(meth)acrylat zum Produkt der Formel V 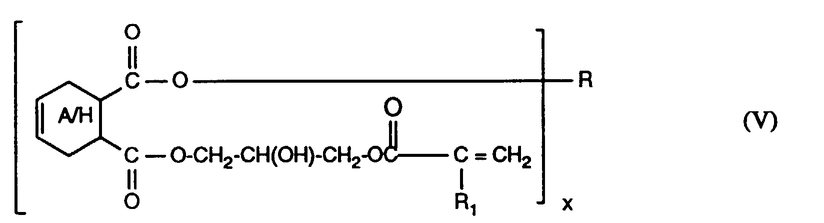 weiter umgesetzt und anschliessend zur (cyclo)aliphatischen Epoxidverbindung der Formel I oxidiert wird, oder, indem man zur Herstellung der Verbindungen der Formel II entsprechende ungesättigte, carboxylhaltige und (Meth)acrylathaltige Verbindungen oxidiert.

Als den Rest R einführende Verbindungen kommen solche in Frage, die mindestens eine Hydroxylgruppe aufweisen, wie 1,4-Butandiol, 1,6-Hexandiol, Diethylenglykol und Polyethylenglykole. Die Umsetzung des Anhydrides der Formel (III) mit der den Rest R einführenden Verbindung erfolgt vorzugsweise bei einer Temperatur von 90°C bis 130°C gegebenenfalls in Anwesenheit eines Katalysators, wie N,N-Dimethylbenzylamin, und gegebenenfalls in einem organischen inerten Lösungsmittel. Die so hergestellte Diester-Disäure der Formel IV muss nicht isoliert werden. Sie wird dann mit Glycidylacrylat/methacrylat in Anwesenheit eines Inhibitors (z.B. Di-tert.-butyl-p-kresol) zur Acrylatverbindung (V) umgesetzt, welche dann z.B. bei einer Temperatur von 15°C bis maximal 40°C oxidiert wird. Die Oxidation erfolgt z.B. mit einer Lösung von Peressigsäure in Essigsäure gegebenenfalls in Anwesenheit eines chlorierten Kohlenwasserstoffes, wie Chloroform oder vorzugsweise Methylenchlorid.

Die offenkettigen (cyclo)aliphatischen Epoxidverbindungen der Formel II können aus ungesättigten carboxylgruppenhaltigen Verbindungen durch Umsetzung der Carboxylgruppen mit Glycidyl(meth)acrylat und anschliessende Oxidation der Doppelbindungen hergestellt werden. Als carboxylgruppenhaltige Verbindungen kommen ungesättigte Disäuren oder ungesättigte Polyether oder Polyester mit sauren Endgruppen in Frage.

Die erfindungsgemässen, neuen (cyclo)aliphatischen Epoxidverbindungen können mit den verschiedensten Komponenten zur Herstellung von Formulierungen weiterverarbeitet werden. Derartige Formulierungen enthalten
a) 5 bis 99 Gew.% einer (cyclo)aliphatischen Epoxidverbindung der Formel I oder II, und
b) 0 bis 10 Gew.% eines radikalischen Photoinitiators.

Typische Verbindungen bekannter Photoinitiatoren sind Benzoine, wie Benzoin, Benzoinether, wie Benzoinmethylether, Benzoinethylether und Benzoinisopropylether, Benzoinphenylether und Benzoinacetat, Acetophenone, wie Acetophenon, 2,2-Dimethoxyacetophenon und l,l-Dichloracetophenon, Benzil, Benzilketale, wie Benzildimethylketal und Benzildiethylketal, Anthrachinone, wie 2-Methylanthrachinon, 2-Ethylanthrachinon, 2-tert.-Butylanthrachinon, 1-Chloranthrachinon und 2-Amylanthrachinon, ferner Triphenylphosphin, Benzoylphosphinoxide, wie beispielsweise 2,4,6-Trimethylbenzoyldiphenylphosphinoxid (Luzirin TPO), Benzophenone, wie Benzophenon und 4,4'-Bis(N,N'-dimethylanino)-benzophenon,Thioxanthone und Xanthone, Acridinderivate, Phenazinderivate, Quinoxalinderivate oder 1-Phenyl-1,2-propandion-2-O-benzoyloxim, 1-Aminophenylketone oder 1-Hydroxyphenylketone, wie l-Hydroxycyclohexylphenylketon, Phenyl-(1-hydroxyisopropyl)-keton und 4-Isopropylphenyl-(1-hydroxyisopropyl)keton, welche alle bekannte Verbindungen darstellen.

Besonders geeignete Photoinitiatoren, welche gewöhnlich in Kombination mit einem He/Cd-Laser als Lichtquelle verwendet werden, sind Acetophenone, wie 2,2-Dialkoxybenzophenone und α-Hydroxy-phenylketone, beispielsweise 1-Hydroxycyclohexylphenylketon oder 2-Hydroxyisopropylphenylketon (= 2-Hydroxy-2,2-dimethylacetophenon).

Eine andere Klasse von Photoinitiatoren, die gewöhnlich bei Verwendung von Argonion-Lasern eingesetzt wird, sind die Benzilketale, wie beispielsweise Benzildimethylketal.

Eine weitere Klasse von geeigneten Photoinitiatoren stellen die ionischen Farbstoff-Gegenionverbindungen (ionic dye-counter ion compound) dar, welche in der Lage sind, aktinische Strahlen zu absorbieren und freie Radikale zu erzeugen, die die Polymerisation der Epoxidverbindungen initiieren können. Die Zusammensetzungen, welche ionische Farbstoff-Gegenionverbindungen enthalten, können auf diese Weise variabler mit sichtbarem Licht im einstellbaren Wellenlängenbereich von 400-700 nm gehärtet werden. Ionische Farbstoff-Gegenionverbindungen und deren Wirkungsweise sind bekannt, beispielsweise aus EP 223 587 und den US-Patenten 4,751,102; 4,772,530 und 4,772,541. Als Beispiele für geeignete ionische Farbstoff-Gegenionverbindungen seien genannt:
die anionischen Farbstoff-Jodoniumionkomplexe, die anionischen Farbstoff-Pyrylliumionkomplexe und insbesondere die kationischen Farbstoff-Boratanionverbindungen der folgenden Formel 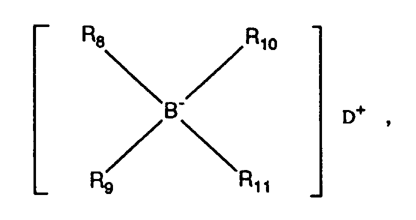 worin D⁺ für einen kationischen Farbstoff steht und R₈, R₉, R₁₀ und R₁₁ unabhängig voneinander je ein Alkyl, Aryl, Alkaryl, Allyl, Aralkyl, Alkenyl, Alkinyl, eine alicyclische oder gesättigte oder ungesättigte heterocyclische Gruppe bedeuten. Bevorzugte Definitionen für die Reste R₈ bis R₁₁ können beispielsweise der EP 223 587 entnommen werden.

Die Photoinitiatoren werden bekanntlich in wirksamen Mengen zugesetzt, das heisst, zweckmässig in Mengen von 0,1 bis 10 Gewichtsprozent, bezogen auf die Gesamtmenge der Zusammensetzung. Wenn die erfindungsgemässen Formulierungen für stereolithographische Verfahren verwendet werden, wobei normalerweise Laserstrahlen eingesetzt werden, ist es wesentlich, dass die Absorptionsfähigkeit der Zusammensetzung durch Typ und Konzentration des Photoinitiators so abgestimmt wird, dass die Härtungstiefe bei normaler Lasergeschwindigkeit ungefähr 0,1 bis 2,5 mm beträgt.

Weitere geeignete Photoinitiatoren können auch Verbindungen sein, welche gegenüber Strahlen von Emissionslinien unterschiedlicher Wellenlängen verschieden strahlungsempfindlich sind. Man erreicht dadurch beispielsweise eine bessere Ausnützung einer UV/VIS-Lichtquelle, welche Emissionslinien unterschiedlicher Wellenlänge ausstrahlt. Vorteilhaft ist es dabei, wenn die verschiedenen Photoinitiatoren so ausgewählt und in einer solchen Konzentration eingesetzt werden, dass bei den verwendeten Emissionslinien eine gleiche optische Absorption erzeugt wird.

Bevorzugte Photoinitiatoren sind α-Hydroxy-phenylketone, insbesondere 1-Hydroxycyclohexyl-phenylketon.

Die oben erwähnten Formulierungen können noch zusätzlich weitere Komponenten enthalten:
c) 0 bis 20 Gew.% übliche Additive, beispielsweise Stabilisatoren, wie UV-Stabilisatoren, Polymerisationsinhibitoren, Trennmittel, Benetzungsmittel, Verlaufsmittel, Sensibilisatoren, Antiabsetzmittel, oberflächenaktive Mittel, Farbstoffe, Pigmente oder Füllstoffe;
d) 0 bis 80 Gew.% eines mono-, di- oder mehrfunktionellen (Meth)-Acrylates, wie Mono(meth)acrylat, Mono-N-Vinylverbindungen mit einem MG von höchstens 500, aliphatische oder cycloaliphatische Di(meth)acrylate, aliphatische Tri(meth)acrylate oder aromatische Di- oder Tri(meth)acrylate;
e) 0 bis 80 Gew.% eines di- oder mehrfunktionellen üblichen aromatischen, alicyclischen oder aliphatischen Epoxidharzes. Epoxidharze, die in den erfindungsgemässen Formulierungen in Frage kommen, sind z.B. in der EP-A-0 360 869 beschrieben. Vorzugsweise werden Butandioldiglycidylether und 3,4-Epoxycyclohexyl-3',4'-epoxycyclohexancarboxylat eingesetzt;
f) 0 bis 50 Gew.% eines OH-terminierten Polyethers oder Polyesters, wie Di- oder trifunktionelle Polyether oder Polyester-Polyole, Polytetrahydrofuran, Poly-ε-caprolacton und OH-terminierte Polyurethane. Von besonderem Interesse ist OH-terminiertes Polycaprolacton;
g) 0,5 bis 5 Gew.% eines kationischen Photoinitiators, wie in der EP-A-0 360 869 beschrieben. Von besonderem Interesse sind Triarylhexafluorantimonat, wie Triarylsulfonium-hexafluoroantimonat.

Bevorzugte Formulierungen enthalten
a) 5 bis 60 Gew.% einer (cyclo)aliphatischen Epoxidverbindung der Formeln I oder II,
b) 0 bis 10 Gew.% eines radikalischen Photoinitiators,
c) 0 bis 10 Gew.% übliche Additive,
d) 0 bis 40 Gew.% eines mono-, di- oder mehrfunktionellen (Meth)-Acrylates,
e) 30 bis 70 Gew.% eines di- oder mehrfunktionellen Epoxides,
f) 5 bis 40 Gew.% eines OH-terminierten Polyethers oder Polyesters, und
g) 0,5 bis 5 Gew.% eines kationischen Photoinitiators.

Die erfindungsgemäss in Frage kommenden Formulierungen können in bekannter Weise hergestellt werden, beispielsweise durch Vormischen einzelner Komponenten und anschliessendes Vermischen dieser Vormischungen oder durch Vermischen aller Komponenten mittels üblicher Vorrichtungen, wie Rührbehälter, zweckmässig in Abwesenheit von Licht und gegebenenfalls bei leicht erhöhter Temperatur, z.B. bei etwa 50 bis 70°C.

Die erfindungsgemäss in Frage kommenden Formulierungen enthaltend die neuen (cyclo)aliphatischen Epoxidverbindungen können durch Bestrahlen mit aktinischem Licht, beispielsweise mittels Elektronen- oder Röntgenstrahlen, UV- oder VIS-Licht, das heisst, mit elektromagnetischer Strahlung oder Teilchenstrahlung im Wellenlängenbereich von 280-650 nm, polymerisiert werden. Besonders geeignet sind Laserstrahlen von He/Cd, Argon- oder Stickstoff- sowie Metalldampf- und NdYAG-Laser mit vervielfachter Frequenz. Es ist dem Fachmann bekannt, dass für jede gewählte Lichtquelle der geeignete Photoinitiator ausgewählt und gegebenenfalls sensibilisiert werden muss. Man hat erkannt, dass die Eindringtiefe der Strahlung in die zu polymerisierende Zusammensetzung und die Arbeitsgeschwindigkeit in direktem Zusammenhang mit dem Absorptionskoeffizienten und der Konzentration des Photoinitiators stehen. In der Stereolithographie werden vorzugsweise solche Photoinitiatoren eingesetzt, welche die grösste Strahlungseindringtiefe in die zu polymerisierenden Zusammensetzungen ermöglichen.

Gegenstand der Erfindung ist somit auch ein Verfahren zum Polymerisieren der erfindungsgemässen Formulierungen, indem man diese mit aktinischem Licht bestrahlt. Die erhaltenen Polymerisate können beispielsweise als Beschichtungsmittel, Photoresists oder Klebstoffe verwendet werden.

Ein weiterer Erfindungsgegenstand ist auch ein Verfahren zur Herstellung von dreidimensionalen Gegenständen aus einer erfindungsgemäss in Frage kommenden Formulierung mittels einem lithographischen Verfahren, insbesondere stereolithographischen Verfahren, wobei die Oberfläche einer Schicht aus der erfindungsgemässen Formulierung ganzflächig oder in einem vorbestimmten Muster mit einer UV und/oder VIS-Lichtquelle bestrahlt wird, so dass sich in den bestrahlten Bereichen eine Schicht in einer gewünschten Schichtdicke verfestigt, dann eine neue Schicht aus der Formulierung auf der verfestigten Schicht gebildet wird, die ebenfalls ganzflächig oder in einem vorbestimmten Muster bestrahlt wird, und wobei durch wiederholtes Beschichten und Bestrahlen dreidimensionale Gegenstände aus mehreren aneinander haftenden, verfestigten Schichten erhalten werden.

Bei diesem Verfahren wird als Lichtquelle vorzugsweise ein Laserstrahl verwendet, der in einer besonders bevorzugten Ausführungsform computergesteuert ist.

Werden die erfindungsgemäss in Frage kommenden Formulierungen als Beschichtungsmittel eingesetzt, so erhält man klare und harte Beschichtungen z.B. auf Holz, Papier, Metall, Keramik oder anderen Oberflächen. Die Beschichtungsdicke kann dabei sehr variieren und etwa von 1 µm bis etwa 1 mm betragen. Aus den erfindungsgemässen Gemischen können Reliefbilder für gedruckte Schaltungen oder Druckplatten direkt durch Bestrahlen der Gemische hergestellt werden, beispielsweise mittels eines computergesteuerten Laserstrahls geeigneter Wellenlänge oder unter Anwendung einer Photomaske und einer entsprechenden Lichtquelle.

Eine weitere Verwendungsmöglichkeit ist der Einsatz der anmeldungsgemässen Formulierungen als photohärtbare Klebstoffe.

Vorzugsweise verwendet man die erfindungsgemässen Formulierungen zur Herstellung von photopolymerisierten Schichten, insbesondere in Form von dreidimensionalen Gegenständen, die aus mehreren aneinander haftenden, verfestigten Schichten aufgebaut sind.

Die folgenden Beispiele veranschaulichen die vorliegende Erfindung weiter.

Beispiel 1: 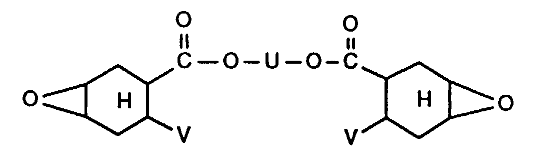 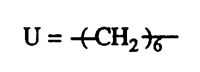 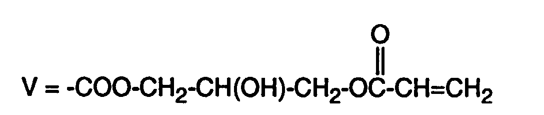 76,07 g (0,5 mol) Tetrahydrophthalsäureanhydrid werden in einem Sulfierkolben mit Rührer, Rückflusskühler und Thermometer bei 110°C geschmolzen. Dann werden 0,67 g N,N-Dimethylbenzylamin und 59,09 g (0,25 mol) 1,6-Hexandiol unter Rühren hinzugefügt. Die Mischung wird 3h bei 110°C gerührt.
Zu der so erhaltenen Diester-Disäure wird 0,27 g Di-tert-butyl-p-kresol hinzugefügt. Dann wird eine Mischung von 64,06 g (0,5 mol) Glycidylacrylat, 0,24 g (0,12 %) Nuosyn Chromium® (Fettsäure-Chromsalz in Kohlenwasserstoffen, erhältlich bei Durham Chemicals, Durham, GB) und 0,13 g Di-tert-butyl-p-kresol zugetropft. Die Mischung wird weiter bei 110°C gerührt, bis ein Epoxidgehalt von <0,05 Aequ./kg erhalten wird (ca. 18h). 180 g des so hergestellten ungesättigten Diacrylats werden in 300 ml Methylenchlorid bei Raumtemperatur gelöst, dann werden 15 g Natriumacetat hinzugefügt. 141,18 g (0,74 mol) 40%-ige Peressigsäure in Essigsäure werden zugetropft, wobei die Temperatur nicht über 40°C steigen soll. Es wird noch 5h bei 35°C nachgerührt.
Die Lösung wird dann mit 5%-igem, wässrigem NaHCO₃ und dann mit Wasser extrahiert, und die organische Phase wird nach dem Trocknen und Zerstören der Peroxide (mit Natriumsulfit) im Vakuum eingedampft.
- Ausbeute:: 142 g (75 %) des Produktes obiger Formel, in Form eines farblosen viskosen Harzes mit einer Viskosität η (20°C)> 10 000 mPa.sec.
- Epoxidgehalt:: 1,51 Aequ./kg (54 % der Theorie).

### Beispiel 2:

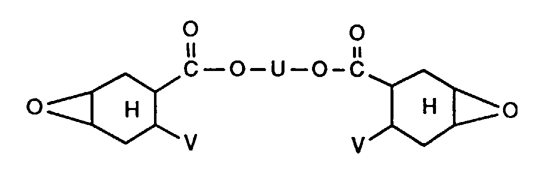

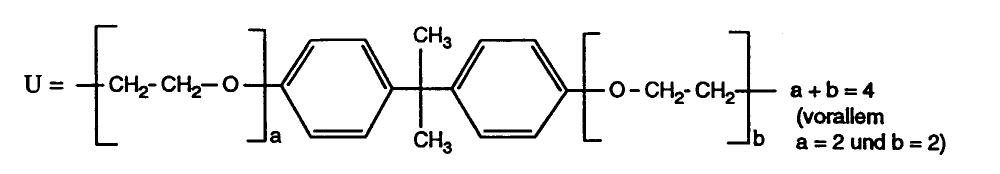

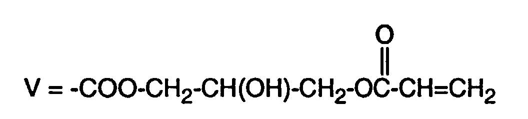

76,07 g (0,5 mol) Tetrahydrophthalsäureanhydrid werden mit 101,1 g (0,25 mol) Dianol 2211 (aliphatisch-aromatischer Dialkohol, erhältlich bei Akzo Chemie; seine chemische Formel entspricht der oben aufgeführten Formel U mit zusätzlich zwei endständigen Hydroxylgruppen) nach der in Beispiel 1 beschriebenen Methode umgesetzt.
Zu der so erhaltenen Diester-Disäure wird eine Mischung von 64,06 g (0,5 mol) Glycidylacrylat, 0,24 g (0,12 %) Nuosyn Chromium® und 0,48 g (0,2 %) Di-tert-butyl-p-kresol zugetropft. Nach einer Reaktionszeit von ca. 5h wird ein Epoxidgehalt von <0,06 Aequ./kg erhalten.
200 g (0,207 mol) des so erhaltenen ungesättigten Diacrylats werden, wie in Beispiel 1 beschrieben, mit 110 g (0,58 mol) 40 %-iger Peressigsäure in Essigsäure oxidiert.
- Ausbeute:: 201,8 g (88 %) des Produktes obiger Formel, in Form eines schwach gelben, viskosen Harzes.
- Epoxidgehalt:: 1,07 Aequ./kg (54 % der Theorie).

### Beispiel 3:

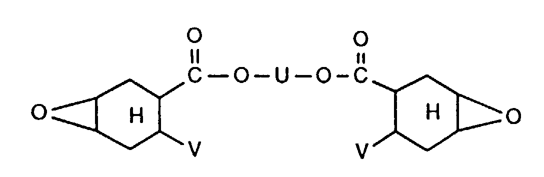

U = -CH₂-CH₂-O-CH₂-CH₂-

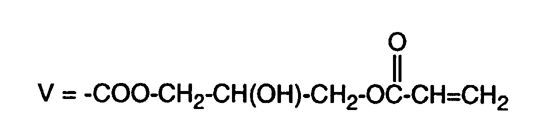

76,07 g (0,5 mol) Tetrahydrophthalsäureanhydrid werden mit 25,5 g (0,25 mol) Diethylenglykol nach der in Beispiel 1 beschriebenen Methode umgesetzt.
Zu der so erhaltenen Diester-Disäure wird eine Mischung von 64 g (0,5 mol) Glycidylacrylat, 0,2 g (0,12 %) Nuosyn Chromium® und 0,33 g (0,2 %) Di-tert-butyl-p-kresol zugetropft. Nach einer Reaktionszeit von 5h wird ein Epoxidgehalt von <0,05 Aequ./kg bestimmt.
50 g (0,075 mol) des so erhaltenen ungesättigten Diacrylats werden, wie in Beispiel 1 beschrieben, mit 39,9 g (0,21 mol) Peressigsäure oxydiert.
- Ausbeute:: 40 g (74,4 %) des Produktes obiger Formel, in Form eines farblosen viskosen Harzes.
- Epoxidgehalt:: 1,89 AequJkg (70,8 % der Theorie).

### Beispiel 4:

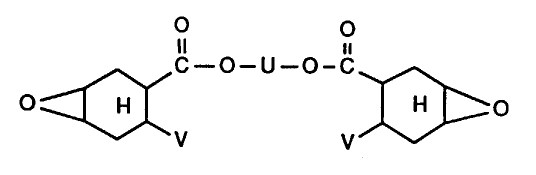

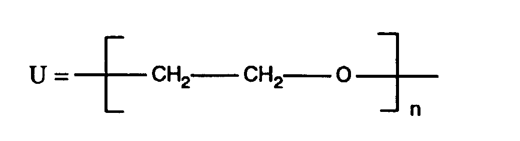

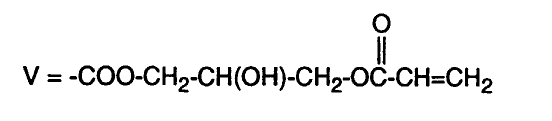

76,07 g (0,5 mol) Tetraydrophthalsäureanhydrid werden mit 150 g (0,25 mol) Polyethylenglykol-600 (n ∼ 14) nach der in Beispiel 1 beschriebenen Methode umgesetzt.
Zu der so erhaltenen Diester-Dicarbonsäure wird eine Mischung von 64 g (0,5 mol) Glycidylacrylat, 0,24 g (0,12 %) Nuosyn Chromium® und 0,58 g (0,2 %) Di-tert-butyl-p-kresol zugetropft. Nach einer Reaktionszeit von 5h bei 120°C wird ein Epoxidgehalt von <0,05 AequJkg bestimmt.
54,27 g (0,045 mol) des so erhaltenen ungesättigten Diacrylats werden mit 23,75 g (0,125 mol) 40 %-iger Peressigsäure in Essigsäure, wie in Beispiel 1 beschrieben, oxidiert.
- Ausbeute:: 50 g (90,8 %) des Produktes obiger Formel, in Form eines farblosen Harzes mit einer Viskosität η = 10 000 mPa.sec (35°C).
- Epoxidgehalt:: 0,76 Aequ./kg (46,2 % der Theorie).

### Beispiel 5:

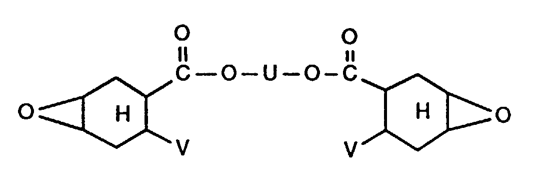

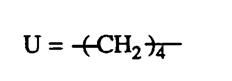

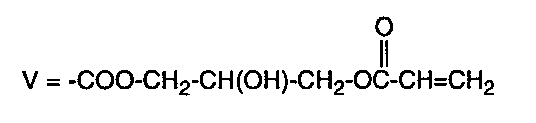

76,07 g (0,5 mol) Tetrahydrophthalsäureanhydrid werden mit 22,53 g (0,25 mol) Butandiol nach der in Beispiel 1 beschriebenen Methode umgesetzt
Zu der so erhaltenen Diester-Disäure wird eine Mischung von 64,06 g (0,5 mol) Glycidylacrylat, 0,2 g (0,12 %) Nuosyn Chromium® und 0,33 g Di-tert.-butyl-p-kresol hinzugetropft. Nach einer Reaktionszeit von ca. 10 h wird ein Epoxidgehalt von <0,06 Aequ./kg erhalten.
70 g (0,11 mol) des so erhaltenen ungesättigten Diacrylats werden, wie in Beispiel 1 beschrieben, mit 59,6 g (0,31 mol) 40 %-iger Peressigsäure in Essigsäure oxidiert.
- Ausbeute:: 56 g (78 %) eines farblosen, viskosen Harzes obiger Formel.
- Expoxidgehalt:: 2,19 Aequ./kg (71,8 % der Theorie).

### Beispiel 6:

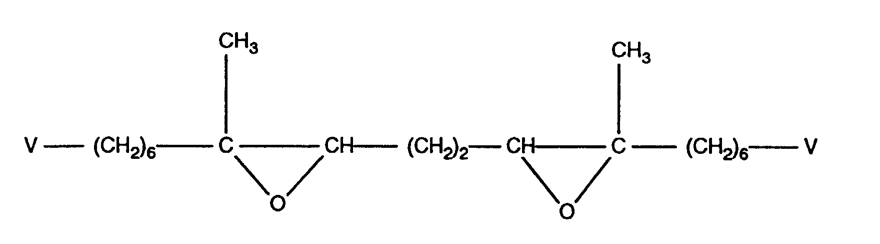

wobei 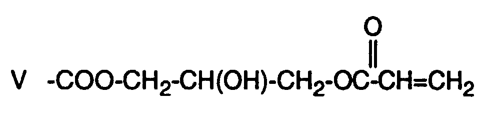 bedeutet.
60 g (0,096 mol) eines Produktes, das durch Umsetzung von IPU 22-G® (ungesättigtes, langkettiges Diepoxid der Firma Okamura Oil Mill, Japan) mit 2 Aequ. Acrylsäure erhalten wird, werden, wie in Beispiel 1 beschrieben, mit 51,3 g (0,27 mol) 40%-iger Peressigsäure in Essigsäure oxidiert.
- Ausbeute:: 59,6 g (95 %) des Produktes obiger Formel, in Form eines farblosen, viskosen Harzes.
- Epoxidgehalt:: 1,1 Aequ./kg (36 % der Theorie).

### Beispiel 7:

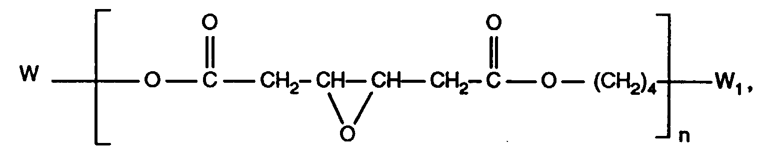

wobei 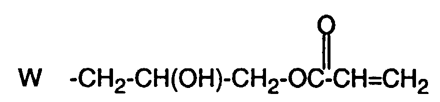 und 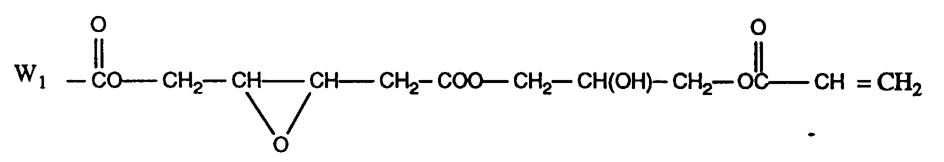 sind.

50 g (0,35 mol) Trans-3-hexendisäure werden mit 15,6 g (0,17 mol) Butandiol in 100 ml Toluol suspendiert. Nach Zugabe von 0,65 g p-Toluolsulfonsäure wird die Mischung auf 120°C erhitzt, wobei das entstehende Wasser mit Hilfe eines Wasserabscheiders entfernt wird. Nach ca. 5h ist die Wasserabscheidung beendet (5,7 ml, Theorie 6,3 ml). Während des Abkühlens bildet sich ein kristalliner Niederschlag, der abfiltriert und aus Tetrahydrofuran umkristallisiert wird.
- Ausbeute:: 28,7 g (49,3 %).
Zu 28,26 g (0,0825 mol) des so hergestellten sauren Polyesters wird eine Mischung von 21,16 g (0,165 mol) Glycidylacrylat, 0,06 g Nuosyn Chromium® und 0,1 g Di-tert-butyl-p-kresol zugetropft. Nach einer Reaktionszeit von 12h wird ein Epoxidgehalt von <0,06 AequJkg erhalten.
30 g (0,05 mol) des so erhaltenen ungesättigten Diacrylats werden, wie in Beispiel 1 beschrieben, mit 53,2 g (0,14 mol) 20%-iger Peressigsäure in Essigsäure oxidiert. Man erhält ein farbloses, viskoses Harz obiger Struktur.
- Epoxidgehalt:: 1,8 Aequ./kg (57 % der Theorie).

### Beispiel 8:

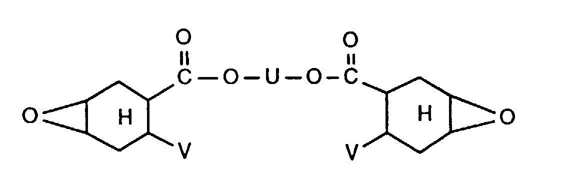

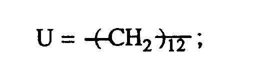

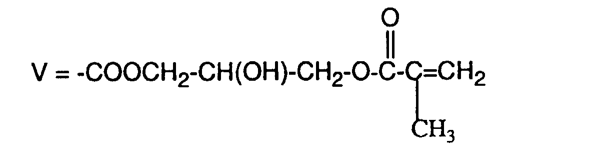

76,07 g (0,5 mol) Tetrahydrophthalsäureanhydrid werden mit 50,59 g (0,25 mol) 1,12-Dodecandiol nach der in Beispiel 1 beschriebenen Methode umgesetzt.
Zu der so erhaltenen Diester-Disäure wird eine Mischung von 71,08 g (0,5 mol) Glycidylmethacrylat, 0,24 g (0,12 % Nuosyn Chromium® und 0,40 g (0,2 %) Di-tert-butyl-p-kresol hinzugetropft. Nach einer Reaktionszeit von 7,5 h wird ein Produkt mit einem Epoxidgehalt von <0,05 Aequ./kg erhalten.
199,02 g (0,25 mol) des so erhaltenen ungesättigten Dimethacrylats werden, wie in Beispiel 1 beschrieben, mit 133,09 g (0,70 mol) 40 %iger Peressigsäure in Essigsäure oxidiert.
- Ausbeute:: 201,6 g (98,2 %) des Produktes obiger Formel, in Form eines braunen, sehr viskosen Harzes.
- Epoxidgehalt:: 1,28 Aequ./kg (52,5 % der Theorie).

### Beispiel 9:

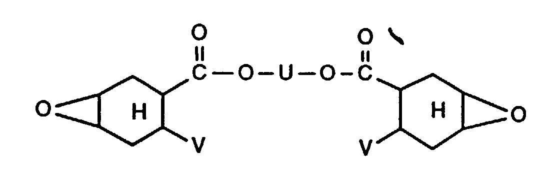

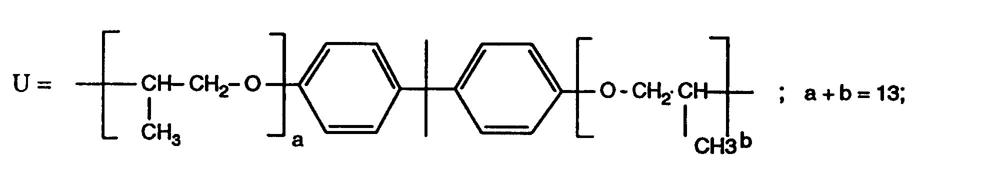

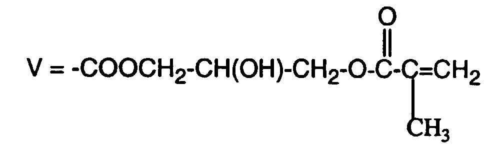

76,07 g (0,5 mol) Tetrahydrophthalsäureanhydrid werden mit 245,84 g (0,25 mol) Dianol® 3310 (aliphatisch-aromatischer Dialkohol, erhältlich bei Akzo Chemie; seine Formel entspricht der oben aufgeführten Formel U mit zusätzlich zwei endständigen Hydroxylgruppen) nach der in Beispiel 1 beschriebenen Methode umgesetzt.
Zu der so erhaltenen Diester-Dicarbonsäure wird eine Mischung von 71,08 g (0,5 mol) Glycidylmethacrylat, 0,47 g (0,12 %) Nuosyn Chromium® und 0,79 g (0,2 %) Di-tert-butyl-p-kresol zugetropft. Nach einer Reaktionszeit von ca. 5,5 h wird ein Produkt mit einem Epoxidgehalt von <0,07 Aequ./kg erhalten.
395 g (0,26 mol) des so erhaltenen ungesättigten Diacrylats werden, wie in Beispiel 1 beschrieben, mit 136,89 g (0,72 mol) 40 %-iger Peressigsäure in Essigsäure oxidiert.
- Ausbeute:: 383,5 g (93,6 %) des Produktes obiger Formel, in Form eines braunen Oels.
- Epoxidgehalt:: 0,72 Aequ./kg (56,7 % der Theorie).

### Beispiel 10: Herstellung der Formulierungen:

Alle im folgenden unter a) bis d) genannten Komponenten werden bei 60°C in einem Rundkolben gemischt, bis eine homogene Lösung entstanden ist. Die Viskositäten dieser Lösungen werden bei 30°C gemessen.
Die mechanischen Eigenschaften der erhaltenen Formulierungen werden an 3-dimensionalen Körpern gemessen, die mit Hilfe eines He/Cd-Lasers hergestellt worden sind, und zwar direkt nach Laserhärtung als auch nach vollständiger Aushärtung (30 Minuten UV, 30 Minuten 130°C).
Der Curl-Faktor (CF) ist ein in der Stereolithographie angewandtes Mass, um Schwundphänomene verschiedener Mischungen miteinander zu vergleichen. Informationen zur Bestimmung des CF sind aus der Literatur zugänglich (z.B. Proceedings 2nd Int. Conference in Rapid Prototyping, Dayton, Ohio, 1991). Die hier angegebenen CF werden an 3-dimensionalen Körpern bestimmt, die eine Schichtdicke von 3 mils (0,076 mm) aufweisen.
a) Die folgenden Komponenten werden bei 60°C in einem Rundkolben gemischt, bis eine klare Lösung erhalten wird:
   55,44 g 3,4-Epoxycyclohexyl-3'4'-epoxycyclohexancarboxylat,
   18,55 g Butandioldiglycidylether,
   13,1 g trifunktionelles OH-terminiertes Polycaprolacton, Union Carbide,
   12,1 g des Produktes gemäss Beispiel 1,
   0,6 g 1-Hydroxycyclohexyl-phenyl-keton, und
   0,6 g Triarylsulfonium-hexafluoroantimonat (Union Carbide; Aryl bedeutet haupsächlich Phenyl).

   Die Viskosität dieser Mischung beträgt 97,2 mPa.sec (30°C).
   Mit Hilfe eines He/Cd-Lasers werden Formkörper hergestellt (320 mJ/cm2). Direkt nach der Laserhärtung (Grünlinge) weisen diese Körper einen E-Modul von 828 N/mm2 und eine Reissdehnung von 5 % auf.
   Zur vollständigen Aushärtung werden die Grünlinge 30 Minuten mit UV-Licht bestrahlt und dann 30 Minuten bei 130°C behandelt. Diese Körper weisen anschliessend folgende Eigenschaften auf: E-Modul 748 N/mm2 und eine Reissdehnung von 22 %.
   CF-Werte betragen 0,01 für CF₆ und CF₁₁.
b) Die folgenden Komponenten werden bei 60°C gemischt, bis eine homogene Lösung erhalten wird:
   55,4 g 3,4-Epoxycyclohexyl-3'4'-epoxycyclohexancarboxylat,
   18 g Butandioldiglycidylether,
   13 g trifunktionelles OH-terminiertes Polycaprolacton, Union Carbide,
   12 g des Produktes gemäss Beispiel 2,
   0,8 g 1-Hydroxycyclohexyl-phenyl-keton, und
   0,8 g Triarylsulfonium-hexafluoroantimonat, Union Carbide.

   Die Viskosität dieser Mischung beträgt 235 mPa.sec (30°C).
   Mit Hilfe eines He/Cd-Lasers werden 3-dimensionale Körper hergestellt.
   Direkt nach der Laserhärtung (320 mJ/cm²) weisen diese Körper einen E-Modul von 338 N/mm² und eine Reissdehnung von 45 % auf.
   Nach vollständiger Aushärtung (30 Minuten UV, 30 Minuten 130°C) beträgt der E-Modul 1563 N/mm² und die Reissdehnung 23 %.
   Die CF-Werte betragen 0,007 (CF₆) und 0,01 (CF₁₁).
c) Die folgenden Komponenten werden bei 60°C gemischt, bis eine homogene Lösung erhalten wird:
   55,4 g 3,4-Epoxycyclohexyl-3'4'-epoxycyclohexancarboxylat,
   18 g Butandioldiglycidylether,
   13 g trifunktionelles OH-terminiertes Polycaprolacton, Union Carbide,
   12 g des Produktes gemäss Beispiel 5,
   0,8 g 1-Hydroxycyclohexyl-phenyl-keton, und
   0,8 g Triarylsulfonium-hexafluoroantimonat, Union Carbide.

   Die Viskosität dieser Mischung beträgt (30°C) 192 mPa.sec.
   Nach der Laserhärtung (640 mJ/cm²) weisen die erhaltenen dreidimensionalen Körper einen E-Modul von 384 N/mm² und eine Reissdehnung von 31 % auf.
   Nach vollständiger Aushärtung (30 min UV, 30 min 130°C) beträgt der E-Modul 1119 N/mm² und die Reissdehnung 12,5 %.
   Der Curl-Faktor eines Formkörpers, der mit dem Baustil "Weave" gebaut worden ist (siehe z.B. Rapid Prototyping and Manufacturing, P.F. Jacobs ed., SME 1992, S. 199 ff), beträgt 0,045 (CF₆).
d) Die folgenden Komponenten werden bei 60°C gemischt, bis eine homogene Lösung erhalten wird:
   47,4 g 3,4-Epoxycyclohexyl-3'4'-epoxycyclohexancarboxylat,
   18 g Butandioldiglycidylether,
   13 g trifunktionelles OH-terminiertes Polycaprolacton, Union Carbide,
   20 g des Produktes gemäss Beispiel 5,
   0,8 g 1-Hydroxycyclohexyl-phenyl-keton, und
   0,8 g Triarylsulfonium-hexafluoroantimonat, Union Carbide.
   Die Viskosität dieser Mischung beträgt 291 mPa.sec. (30°C).
   Nach der Laserhärtung (640 mJ/cm²) weisen die erhaltenen dreidimensionalen Körper einen E-Modul von 68,8 N/mm² und eine Reissdehnung von 39 % auf.
   Nach vollständiger Aushärtung (30 min UV, 30 min 130°C) beträgt der E-Modul 587 N/mm² und die Reissdehnung 48 %.

Der Curl-Faktor eines Formkörpers, der mit dem Baustil "Weave" gebaut worden ist, beträgt 0,01.

## Patentansprüche

1. (Cyclo)aliphatische Epoxidverbindungen der Formeln I oder II 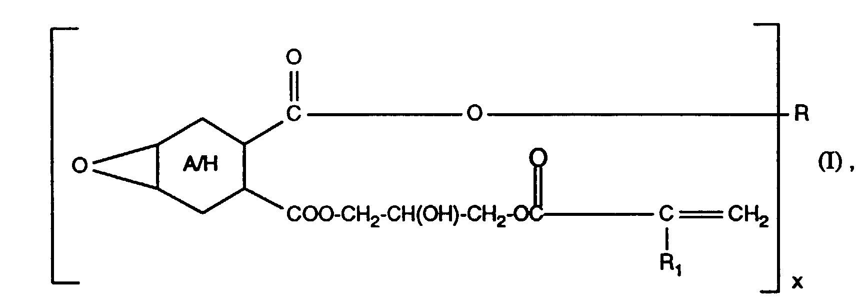 und 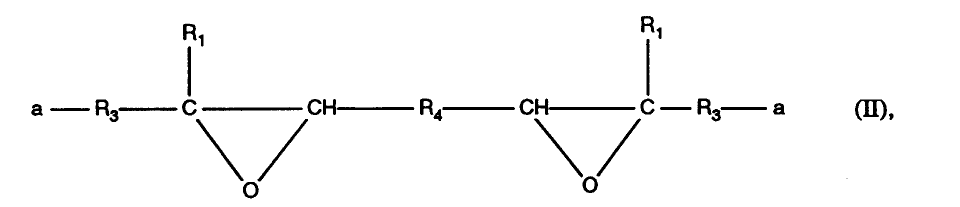 worin
a einen Rest der Formel 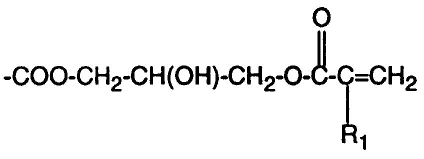 bedeutet,
A einen hydrierten Benzolrest oder einen hydrierten Rest der Formel 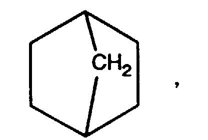 R einen Rest der Formeln (-CH₂)_{y}CH₃, (-CH₂-)_{y}, 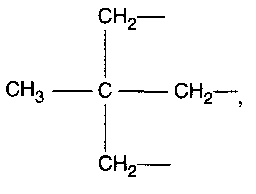 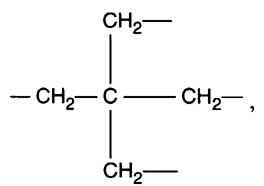 einen unsubstituierten oder substituierten 1 bis 6-wertigen aliphatischen Alkoholrest, einen unsubstituierten oder substituierten aromatisch-aliphatischen Alkoholrest, einen unsubstituierten oder substituierten 1- bis 4-wertigen Polyetherrest oder Polyesterrest, einen unsubstituierten oder substituierten 1- bis 4-wertigen Polycaprolactonrest, einen unsubstituierten oder substituierten 1- bis 4-wertigen Polyurethanrest oder einen Rest der Formel 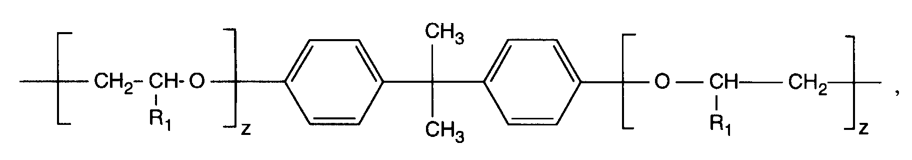 und R₁ Wasserstoff oder CH₃ bedeuten, wobei x eine ganze Zahl von 1 bis 6, y eine ganze Zahl von 2 bis 20, z eine ganze Zahl von 1 bis 10, und
R₃ und R₄ unabhängig voneinander einen unsubstituierten oder substituierten aliphatischen, aromatischen oder cycloaliphatischen Kohlenwasserstoffrest bedeuten, wobei es sich im Falle eines unsubstituierten oder substituierten aliphatischen Kohlenwasserstoffrests um eine gerade oder verzweigte C₁-C₂₀-Alkylenkette handelt, welche ein- oder mehrmals substituiert sein kann und welche auch durch Ester- oder Ether-Gruppierungen unterbrochen sein kann.

2. (Cyclo)aliphatische Epoxidverbindungen gemäss Anspruch 1, dadurch gekennzeichnet, dass diese der Formel I entsprechen.

3. (Cyclo)aliphatische Epoxidverbindungen der Formel 1 gemäss Anspruch 1, dadurch gekennzeichnet, dass
A einen hydrierten Benzolrest, den Rest R (-CH₂-)_{y} oder einen Rest der Formel 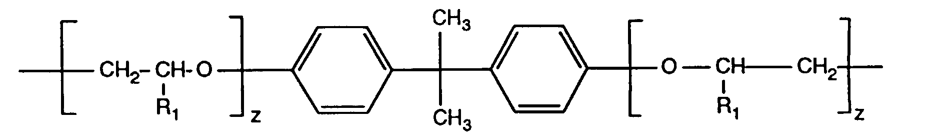 bedeutet, wobei R₁, y und z die im Anspruch 1 unter Formel I angegebene Bedeutung haben, und x 2 bedeutet.

4. Verfahren zur Herstellung der (cyclo)aliphatischen Epoxidverbindungen gemäss Anspruch 1, dadurch gekennzeichnet, dass mindestens 1 Mol eines cyclischen, ungesättigten Anhydrides der Formeln III 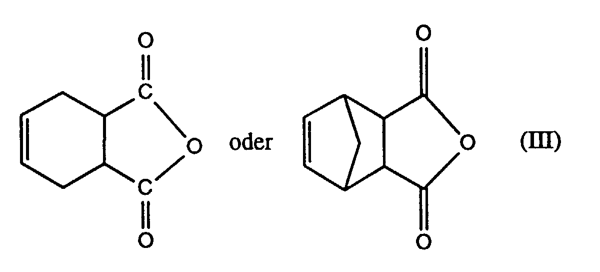 mit mindestens 1 Mol einer den Rest R einführenden Verbindung zu einer Diester-Disäure der Formel IV 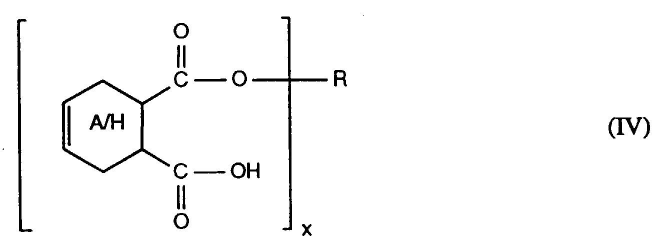 umgesetzt wird, welche dann mit Glycidyl(meth)acrylat zum Produkt der Formel V 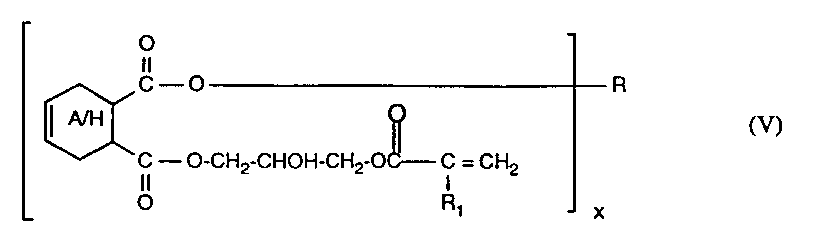 weiter umgesetzt und anschliessend zur (cyclo)aliphatischen Epoxidverbindung der Formel I oxidiert wird, oder, indem man zur Herstellung der Verbindungen der Formel II gemäss Anspruch 1 entsprechende ungesättigte, carboxylhaltige und (Meth)acrylathaltige Verbindungen oxidiert, wobei x, R und R₁ die im Anspruch 1 angegebene Bedeutung haben.

5. Zusammensetzungen enthaltend:
a) 5 bis 60 Gew.% einer (cyclo)aliphatischen Epoxidverbindung der Formeln I oder II gemäss Anspruch 1,
b) 0 bis 10 Gew.% eines radikalischen Photoinitiators,
c) 0 bis 10 Gew.% übliche Additive,
d) 0 bis 40 Gew.% eines mono-, di- oder mehrfunktionellen (Meth)-Acrylates,
e) 30 bis 70 Gew.% eines di- oder mehrfunktionellen Epoxides,
f) 5 bis 40 Gew.% eines OH-terminierten Polyethers oder Polyesters, und
g) 0,5 bis 5 Gew.% eines kationischen Photoinitiators.

6. Verwendung der Zusammensetzungen gemäss Anspruch 5 zur Herstellung von Beschichtungsmitteln, Klebstoffen, Photoresists oder zur Verwendung in der Stereolithographie.

7. Verfahren zum Polymerisieren der Zusammensetzungen gemäss Anspruch 5, indem man diese mit aktinischem Licht bestrahlt.

8. Verfahren zur Herstellung von dreidimensionalen Gegenständen aus einer Zusammensetzung gemäss Anspruch 5 mittels einem lithographischen Verfahren, wobei die Oberfläche einer Schicht der besagten Zusammensetzung ganzflächig oder in einem vorbestimmten Muster mit einer UV/- und/oder VIS-Lichtquelle bestrahlt wird, so dass sich in den bestrahlten Bereichen eine Schicht in einer gewünschten Schichtdicke verfestigt, dann eine neue Schicht dieser Zusammensetzung auf der verfestigten Schicht gebildet wird, die ebenfalls ganzflächig oder in einem vorbestimmten Muster bestrahlt wird, und wobei durch wiederholtes Beschichten und Bestrahlen dreidimensionale Gegenstände aus mehreren aneinander haftenden, verfestigten Schichten erhalten werden.

9. Verfahren gemäss Anspruch 8, wonach als Lichtquelle ein Laserstrahl, vorzugsweise ein computergesteuerter Laserstrahl, verwendet wird.

## Claims

1. A (cyclo)aliphatic epoxy compound of formula I or II 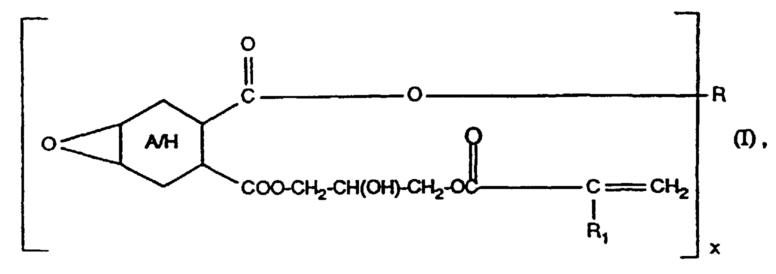 and 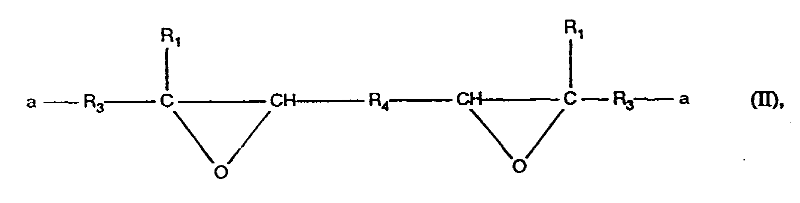 wherein a is a radical of formula 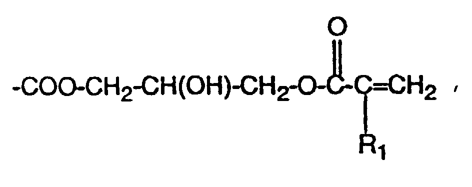 A is a hydrogenated benzene radical or a hydrogenated radical of formula 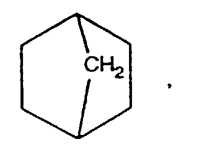 R is a radical of formula (-CH₂)_{y}CH₃, (-CH₂-)_{y}, 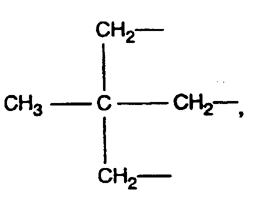 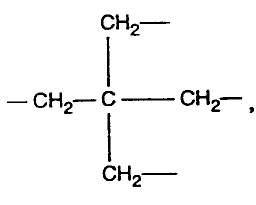 an unsubstituted or substituted monovalent to hexavalent aliphatic alcohol radical, an unsubstituted or substituted aromatic-aliphatic alcohol radical, an unsubstituted or substituted monovalent to tetravalent polyether radical or polyester radical, an unsubstituted or substituted monovalent to tetravalent polycaprolactone radical, an unsubstituted or substituted monovalent to tetravalent polyurethane radical or a radical of formula 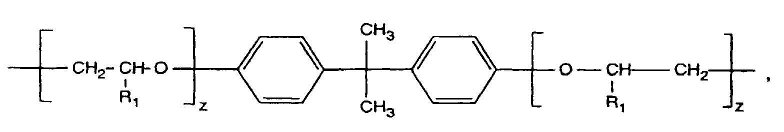 and R₁ is hydrogen or CH₃, x is an integer from 1 to 6, y is an integer from 2 to 20, z is an integer from 1 to 10, and
R₃ and R₄ are each independently of the other an unsubstituted or substituted aliphatic, aromatic or cycloaliphatic hydrocarbon radical, where, in the case of an unsubstituted or substituted aliphatic hydrocarbon radical, the chain concerned is a straight or branched C₁-C₂₀alkylene chain which can be substituted one or more times and which can also be interrupted by ester groups or ether groups.

2. A (cyclo)aliphatic epoxy compound according to claim 1 of formula I.

3. A (cyclo)aliphatic epoxy compound of formula I according to claim 1, wherein
A is a hydrogenated benzene radical, the radical R is (-CH₂-)_{y} or a radical of formula 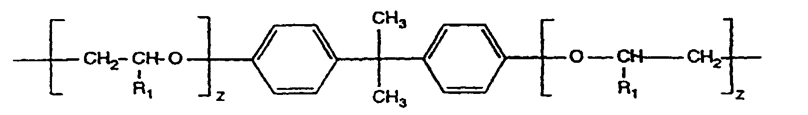 where R₁, y and z are as defined in claim 1 for formula I, and x is 2.

4. A process for the preparation of a (cyclo)aliphatic epoxy compound according to claim 1, which comprises reacting at least 1 mol of a cyclic unsaturated anhydride of formulae III 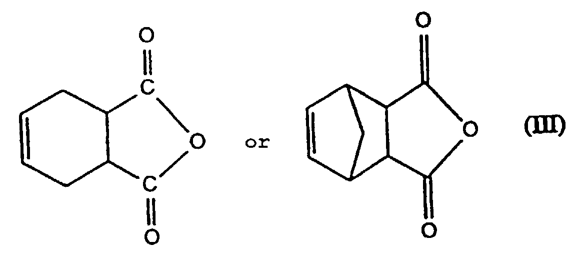 with at least 1 mol of a compound that introduces the radical R to give a diester-diacid of formula IV 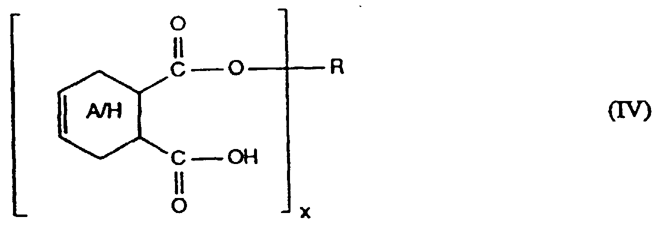 which is then further reacted with glycidyl(meth)acrylate to give the product of formula V 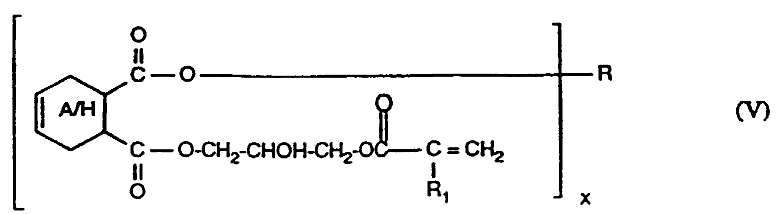 which is then oxidised to give the (cyclo)aliphatic epoxy compound of formula I, or, to prepare a compound of formula II according to claim 1, oxidising a corresponding unsaturated carboxyl-containing and (meth)acrylate-containing compound, where x, R and R₁ are as defined in claim 1.

5. A formulation comprising:
a) 5 to 60% by weight of a (cyclo)aliphatic epoxy compound of formula I or II according to claim 1,
b) 0 to 10% by weight of a radical photoinitiator,
c) 0 to 10% by weight of customary additives,
d) 0 to 40% by weight of a mono-, di- or polyfunctional (meth)acrylate,
e) 30 to 70% by weight of a di- or polyfunctional epoxide,
f) 5 to 40% by weight of an OH-terminated polyether or polyester, and
g) 0.5 to 5% by weight of a cationic photoinitiator.

6. The use of a formulation according to claim 5 for the production of coating compositions, adhesives, photoresists or for use in stereolithography.

7. A process for polymerising a formulation according to claim 5, which comprises irradiating said formulation with actinic light.

8. A process for the production of a three-dimensional object from a formulation according to claim 5 by a lithographic technique, which comprises irradiating the surface of a layer of said formulation over the entire surface or in a predetermined pattern with a UV and/or VIS light source, such that within the irradiated areas a layer solidifies in a desired layer thickness, then forming a new layer of said formulation on the solidified layer, which is likewise irradiated over the entire surface or in a predetermined pattern, such that by repeated coating and irradiation a three-dimensional object is formed from a plurality of solidified layers which adhere to one another.

9. A process according to claim 8, wherein a laser beam, preferably a computer-controlled laser beam, is used as light source.

## Revendications

1. Composés époxydes (cyclo)aliphatiques ayant les formules I ou II 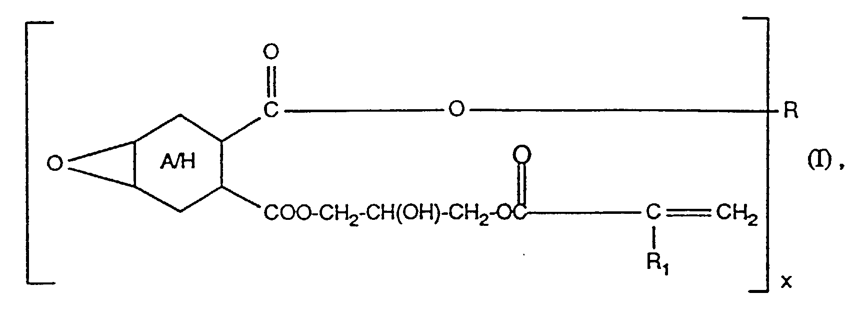 et 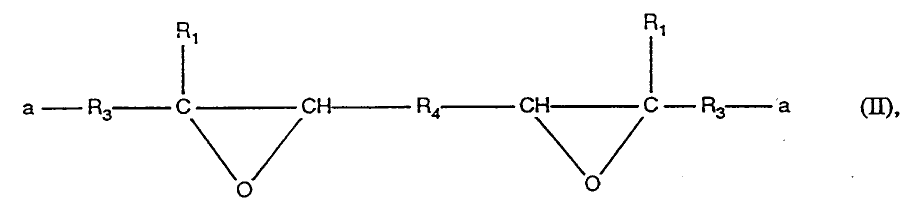 où
a est un radical de formule 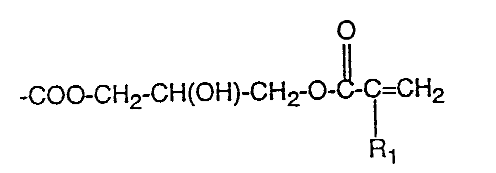 A est un résidu benzénique hydrogéné ou un résidu hydrogéné de formule 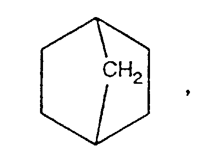 R est un radical de formule
(-CH₂)_{y}CH₃, (-CH₂-)_{y}, 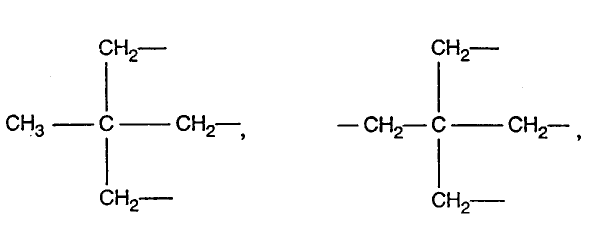 un résidu alcool aliphatique monovalent à hexavalent, non-substitué ou substitué, un résidu alcool aromatique-aliphatique non-substitué ou substitué, un résidu polyéther ou polyester monovalent à tétravalent non-substitué ou substitué, un résidu polycaprolactone monovalent à tétravalent non-substitué ou substitué, un résidu polyuréthanne monovalent à tétravalent non-substitué ou substitué, ou un radical de formule 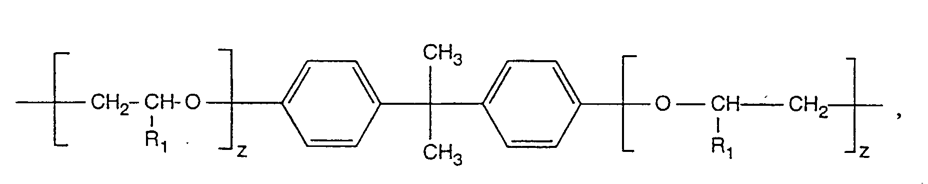 et
R₁ est un hydrogène ou CH₃,
x est un nombre entier de 1 à 6,
y est un nombre entier de 2 à 20,
z est un nombre entier de 1 à 10, et
R₃ et R₄, indépendamment l'un de l'autre, sont chacun un radical hydrocarboné aliphatique, aromatique ou cycloaliphatique, non-substitué ou substitué,
où, dans le cas d'un radical hydrocarboné aliphatique non-substitué ou substitué, il s'agit d'une chaîne alkylène en C₁-C₂₀ linéaire ou ramifiée, qui peut être une ou plusieurs fois substitué et qui peut aussi être interrompue par des groupements ester ou éther.

2. Composés époxydes (cyclo)aliphatiques selon la revendication 1, caractérisés en ce qu'ils correspondent à la formule I.

3. Composés époxydes (cyclo)aliphatiques de formule I selon la revendication 1, caractérisés en ce que A est un résidu benzénique hydrogéné, le radical R est (-CH₂-)_{y} ou un résidu de formule 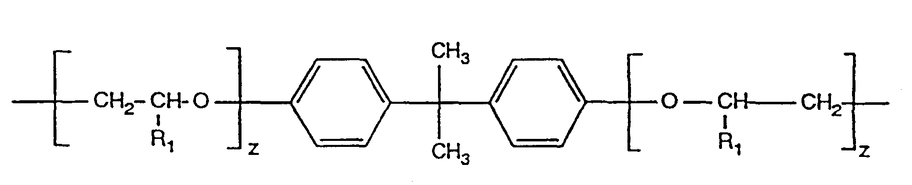 dans laquelle R₁, y et z ont les significations données dans la revendication 1 à propos de la formule I et x vaut 2.

4. Procédé pour préparer les composés époxydes (cyclo)aliphatiques selon la revendication 1, caractérisé en ce qu'on fait réagir au moins 1 mole d'un anhydride insaturé cyclique ayant les formules III 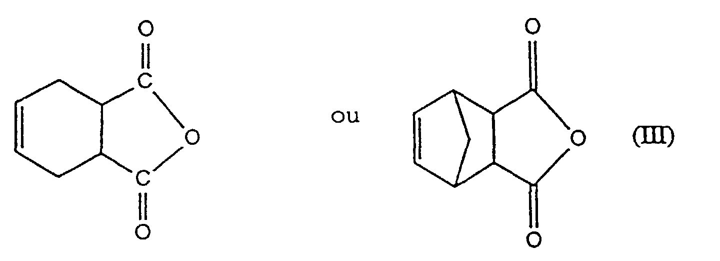 avec au moins 1 mole d'un composé introduisant le radical R, pour obtenir un diester-diacide de formule IV 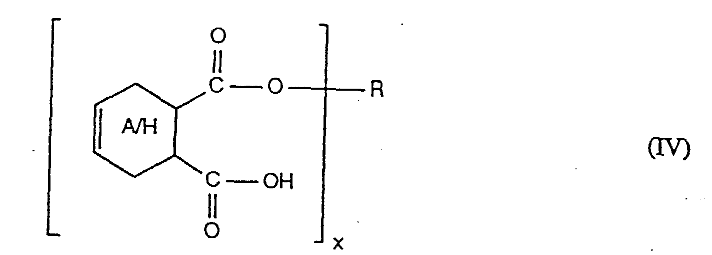 qui ensuite est encore mis à réagir avec du (méth)acrylate de glycidyle pour donner le produit de formule V 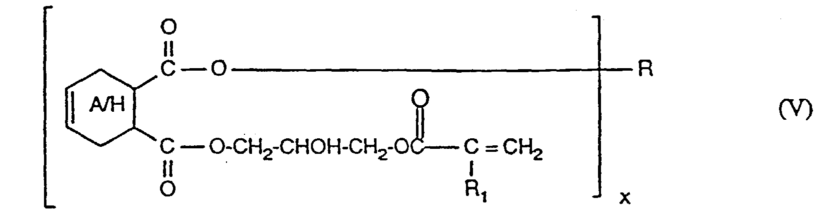 puis on l'oxyde en le composé époxyde (cyclo)aliphatique de formule I, ou, pour préparer les composés de formule II selon la revendication 1, en oxydant des composés insaturés carboxylés et contenant des groupes (méth)acrylates correspondants, où x, R et R₁ ont les significations données dans la revendication 1.

5. Compositions contenant
a) de 5 à 60 % en poids d'un composé époxyde (cyclo)aliphatique ayant les formules I ou II selon la revendication 1
b) de 0 à 10 % en poids d'un photo-amorceur radicalaire,
c) de 0 à 10 % en poids d'additifs usuels,
d) de 0 à 40 % en poids d'un (méth)acrylate mono-, di- ou polyfonctionnel,
e) de 30 à 70 % en poids d'un époxyde di- ou polyfonctionnel,
f) de 5 à 40 % en poids d'un polyéther ou polyester à groupe OH terminal, et
g) de 0,5 à 5 % en poids d'un photo-amorceur cationique.

6. Utilisation des compositions selon la revendication 5 pour produire des agents de revêtement, des adhésifs, des photoréserves, ou pour utilisation en stéréolithographie.

7. Procédé pour polymériser les compositions selon la revendication 5, qui consiste à les exposer à une lumière actinique.

8. Procédé pour fabriquer des objets tridimensionnels à partir d'une composition selon la revendication 5 par un procédé lithographique, dans lequel la surface d'une couche ayant la composition donnée est, en pleine surface ou selon un modèle prédéfini, exposée à une source de lumière UV et/ou VIS, de façon qu'une couche se consolide dans les zones irradiées avec une épaisseur de couche souhaitée, puis on forme une nouvelle couche ayant cette composition sur la couche consolidée, qui elle aussi est irradiée en pleine surface ou selon un modèle prédéterminé, en répétant des opérations de revêtement et d'irradiation pour obtenir des objets tridimensionnels à partir de plusieurs couches consolidées qui adhèrent les unes aux autres.

9. Procédé selon la revendication 8, dans lequel on utilise en tant que source de lumière un faisceau laser, de préférence un faisceau laser commandé par ordinateur.
